# EUROPÄISCHE PATENTSCHRIFT

(11) **EP 2 155 091 B1**
(45) Veröffentlichungstag und Bekanntmachung des Hinweises auf die Patenterteilung: **24.10.2012**
(21) Anmeldenummer: 08749427.4
(22) Anmeldetag: 09.05.2008
(51) Int. Cl.: A61B 18/12, A61B 18/16, A61B 19/00

(54) **HF-CHIRURGISCHE PRÜFEINRICHTUNG**
HF SURGICAL TESTING DEVICE
DISPOSITIF D'ESSAI CHIRURGICAL À HAUTE FRÉQUENCE (HF)

(30) Priorität: 14.05.2007 DE 102007022548; 27.11.2007 DE 102007056974
(43) Veröffentlichungstag der Anmeldung: 24.02.2010
(73) Patentinhaber: ERBE Elektromedizin GmbH, 72072 Tübingen (DE)
(72) Erfinder: SELIG, Peter, 72379 Hechingen (DE)
(74) Vertreter: Bohnenberger, Johannes
(86) Internationale Anmeldenummer: PCT/EP2008/003770
(87) Internationale Veröffentlichungsnummer: WO 2008/138566

(56) Entgegenhaltungen:
- EP-A- 0 836 868
- US-A- 6 125 298

## Beschreibung

Die Erfindung betrifft eine HF-chirurgische Prüfeinrichtung zum Prüfen einer Neutralelektrode beim Behandeln, insbesondere monopolaren Koagulieren biologischen Gewebes mittels eines hochfrequenten Stromes.

Die Hochfrequenzchirurgie wird seit vielen Jahren sowohl in der Human- als auch in der Veterinärmedizin eingesetzt, um biologisches Gewebe z. B. zu koagulieren und/oder zu schneiden. Dabei wird mit Hilfe geeigneter elektrochirurgischer Instrumente hochfrequenter Strom durch das zu behandelnde Gewebe geleitet, so dass sich dieses aufgrund Eiweißkoagulation und Dehydratation verändert. Das Gewebe zieht sich dabei derart zusammen, dass die Gefäße verschlossen und Blutungen gestillt werden. Eine darauf folgende Erhöhung der Stromdichte bewirkt ein explosionsartiges Verdampfen der Gewebeflüssigkeit und ein Aufreißen der Zellmembranen, wobei das Gewebe vollständig durchtrennt wird.

Für die thermische Behandlung biologischen Gewebes werden sowohl bipolare, als auch monopolare Techniken angewendet. Bei monopolaren Anordnungen wird der von dem HF-Generator an das elektrochirurgische Instrument zugeführte HF-Strom in das zu behandelnde Gewebe über eine differente Elektrode appliziert, wobei der Strompfad durch den Körper eines Patienten bis zu einer indifferenten Neutralelektrode führt und von dort zurück zum HF-Generator. An der differenten Elektrode ist eine hohe Stromdichte pro Flächeneinheit zur Behandlung vorgesehen, während bei der indifferenten Elektrode die Stromdichte pro Flächeneinheit gegenüber der differenten Elektrode deutlich verringert ist. Dies lässt sich durch ein entsprechend großflächiges Auslegen der Neutralelektrode erreichen. Nur so ist gewährleistet, dass an dem Gewebe beim Übergang zwischen Gewebe und Neutralelektrode keine Verletzungen, wie z. B. Verbrennungen auftreten.

Zur Durchführung einer Koagulation werden HF-Chirurgieeinrichtungen verwendet, die ein HF-Chirurgiegerät mit einem HF-Generator zur Erzeugung einer hochfrequenten Spannung bzw. eines hochfrequenten Wechselstromes aufweisen, sowie Schalteinrichtungen und/oder Steuer- und Regeleinrichtung zur Aktivierung bzw. Deaktivierung bzw. ganz allgemein zur Steuerung des HF-Generators.

Zur Sicherheit des Patienten sollte es vorgesehen sein, während eines Eingriffs stets zu überprüfen, ob die Neutralelektrode funktionsgerecht arbeitet und z. B. ordnungsgemäß am Patienten anliegt. Jegliches Ablösen der Elektrode führt zu einer gefährlichen Erhöhung der Stromdichte an den noch anhaftenden Bereichen, so dass ggf. oben beschriebene Verletzungen auftreten können. Um eine hohe Sicherheit für den Patienten zu gewährleisten, werden Überwachungsschaltungen eingesetzt, die z. B. das Anhaften der Neutralelektrode am Patienten überprüfen. Derartige Neutralelektroden-Überwachungsschaltungen von HF-Chirurgieeinrichtungen ermitteln in der Regel den Übergangswiderstand und/oder die Stromverteilung zwischen den beiden leitfähigen Abschnitten der Neutralelektrode. Häufig werden aus den Messwerten auch Rückschlüsse auf eine mögliche Erwärmung der Elektroden gezogen. Diese sind allerdings nur dann zutreffend, wenn elektrodenspezifische Parameter, wie z. B. Fläche, Geometrie und Aufbau der Elektrode bekannt sind. Besonders problematisch ist hier die Beurteilung sehr kleiner Neutralelektroden, wie sie für Säuglinge und Kleinkinder angeboten werden. Diese dürfen nur mit einer reduzierten HF-Stromstärke betrieben werden, da ansonsten die Erwärmung unzulässig hohe Werte erreichen kann. Problematisch ist es hier also, die Neutralelektroden bzgl. ihres "Betriebsverhaltens" zu beurteilen.

Aus den Druckschriften US 6,125,298 und EP 0 836 868 A2 ist eine Kodierung zur Erkennung des Elektrodentyps für Defibrillationselektroden bekannt.

Die Aufgabe der vorliegenden Erfindung besteht folglich darin, eine HF-chirurgische Prüfeinrichtung vorzuschlagen, die diese Beurteilung ermöglicht und ein hohes Maß an Sicherheit sowohl für den Patienten als auch für den Operateur bei Verwendung einer Neutralelektrode gewährleistet und dabei möglichst einfach in der Anwendung ist. Diese Aufgabe wird durch eine HF-chirurgische Prüfeinrichtung nach Patentanspruch 1 gelöst. Vorteilhafte Weiterbildungen sind in den Unteransprüchen angegeben.

Insbesondere wird die Aufgabe durch eine HF-chirurgische Prüfeinrichtung zum Prüfen einer Neutralelektrode beim Behandeln, insbesondere monopolaren Koagulieren biologischen Gewebes mittels eines hochfrequenten Stromes gelöst, wobei die Neutralelektrode mindestens einen ersten, mit dem Gewebe in Kontakt bringbaren elektrisch leitfähigen Elektrodenabschnitt mit einer ersten Leitung zum Verbinden mit einem HF-Generator und einen zweiten, mit dem Gewebe in Kontakt bringbaren elektrisch leitfähigen Elektrodenabschnitt mit einer zweiten Leitung zum Verbinden mit dem HF-Generator umfasst und wobei die Prüfeinrichtung ein Kodierungselement mit einer die Neutralelektrode charakterisierenden Kodierung zwischen dem ersten und dem zweiten Elektrodenabschnitt und eine Messeinrichtung umfasst, die derart ausgebildet ist, dass die Kodierung zur Erkennung der Neutralelektrode erfassbar ist.

Ein wesentlicher Punkt der Erfindung liegt darin dass ohne großen Aufwand eine Elektrodenerkennung geteilter Neutralelektroden durchführbar ist, die es erlaubt, den Behandlungsablauf planbarer zu gestalten. So kann durch die Erkennung der Neutralelektrode, d. h. durch die Erkennung des Typs der Neutralelektrode, ein optimierter, insbesondere ein eine hohe Sicherheit für den Patienten gewährleistender Behandlungsablauf vorgenommen werden. Zum Beispiel sind aufgrund der erkannten Neutralelektrode bestimmte Strom- und/oder Spannungswerte gezielt einstellbar.

Vorzugsweise umfasst die Prüfeinrichtung bzw. die Messeinrichtung eine Quelle für Gleichstrom oder niederfrequenten Wechselstrom als ein Prüfstrom, zur Erfassung der Kodierung des Kodierungselements. Die elektrische und/oder elektronische Dekodierung lässt sich ohne großen Aufwand durchführen. Hierbei wird eine Materialeigenschaft des verwendeten Hydrogels zum Befestigen der Neutralelektrode am Patienten genutzt. Das Hydrogel weist eine chemische Zusammensetzung derart auf, dass es für Gleichstrom und Wechselstrom mit sehr niedrigen Frequenzen (bis ca. 100 Hz) eine sehr geringe elektrische Leitfähigkeit aufweist, also hochohmig ist. Wird nun auf der geteilten Neutralelektrode zwischen die beiden Teilflächen ein Kodierungselement eingebaut, so kann dieses mit dem Gleichstromsignal oder dem Wechselstromsignal mit sehr geringer Frequenz ausgemessen werden. Da sich das Gel in diesem Zustand hochohmig verhält, spielt es keine Rolle, ob die Neutralelektrode am Patienten angelegt ist, d. h. ein niederohmiger Parallelwiderstand durch das Gewebe vorhanden ist oder nicht.

Nach einem bevorzugten Aspekt der vorliegenden Erfindung ist die Prüfeinrichtung bzw. sind mindestens Teile der Prüfeinrichtung derart zwischen der ersten Leitung und der zweiten Leitung angeordnet, dass der Prüfstrom über die erste und zweite Leitung führbar ist. Da der Prüfstrom - anders als der Arbeitsstrom - ein Gleichstrom oder ein niederfrequenter Wechselstrom ist, ist die Erfassung der charakterisierenden Kodierung möglich, ohne eine dafür gesonderte Leitungsführung vorzusehen. Das vorhandene Leitungssystem dient also gleichzeitig als Leitungssystem für die Messeinrichtung und damit für die Erfassung der Kodierung. Eine zusätzliche Prüfleitung oder Messleitung ist somit nicht erforderlich. Das heißt, ein erheblicher Vorteil bzgl. Kompatibilität ergibt sich dadurch, dass trotz des erweiterten Funktionsumfangs der Neutralelektrodenerkennung nur die beiden vorhandenen Neutralelektroden-Anschlüsse zur Messung verwendet werden. Damit bleiben sowohl die Elektroden, Anschlusskabel und die Steckverbinder kompatibel zu den am Markt verbreiteten Komponenten. Es werden keine zusätzlichen Leitungen oder elektrischen Kontakte benötigt.

In einer bevorzugten Ausführungsform ist es vorgesehen, dass das Kodierungselement ein Widerstandselement mit einem die Neutralelektrode charakterisierenden Widerstandswert umfasst, wobei die Prüfeinrichtung derart ausgebildet ist, dass der Widerstandswert zur Erkennung der Neutralelektrode erfassbar ist. Die Kodierung über ein Widerstandselement lässt sich einfach bewerkstelligen und mittels des Prüfstroms einfach erkennen.

Das Widerstandselement ist vorzugsweise als ein ohmscher Widerstand oder ein komplexer Widerstand mit induktivem Verhalten ausgebildet. Die verwendeten Widerstände (Kodierungswiderstände) müssen Widerstandswerte aufweisen, die wesentlich kleiner sind als der Widerstand des Gels bei der Messfrequenz. Allerdings müssen die Werte wiederum so groß sein, dass keine nennenswerten HF-Ströme über den Widerstand zwischen den beiden Elektrodenabschnitten fließen können. Typische Werte bewegen sich im Bereich 1kΩ < R_{K} < 100kΩ. Für Gleichstrom und Wechselstrom mit niedriger Frequenz gilt: R_{Gel} > R_{K} > Rp.

In einer bevorzugten Ausführungsform ist das Widerstandselement als eine in die Neutralelektrode integrierte Widerstandsfolie oder ein Widerstandsdraht vorgesehen. Damit ist es möglich, auch Elektroden ohne feste Kabel, die über ein entsprechendes Kabel mit einer Klammer kontaktiert werden, uneingeschränkt mit dieser Funktionalität auszustatten. Das Widerstandselement ist also z. B. auf der geteilten Neutralelektrode zwischen den beiden Elektrodenabschnitten eingebaut.

Bei Elektroden, die mit entsprechenden Leitungen ausgebildet sind, kann das Kodierungselement bzw. das Widerstandselement oder ggf. können die Widerstandselemente zwischen der ersten Leitung und der zweiten Leitung angeordnet sein. Das Anbringen eines Widerstandes z. B. an den Leitungsabschnitten ist sehr einfach realisierbar.

Das Entkoppeln des Prüfstroms vom (hochfrequenten) Arbeitsstrom kann z. B. durch die Zuschaltung einer Induktivität als Filterelement in das Messsystem vorgenommen werden.

In einer Ausgestaltung weist die Prüfeinrichtung bzw. die Messeinrichtung eine Spannungsmesseinrichtung zur Messung einer über dem Kodierungselement bzw. Widerstandselement abfallenden Spannung auf (z. B. aufgrund des Prüfstroms). Somit lässt sich auf einfache Weise der Widerstandswert für die entsprechende Neutralelektrode bestimmen. Es ist auch möglich, die Kodierung bzw. den Widerstandswert über eine Strommesseinrichtung zur Messung des Gleichstroms oder des niederfrequenten Stroms zu bestimmen. Diese Art der indirekten Messung kann unproblematisch bewerkstelligt werden.

In einer Ausgestaltung der erfindungsgemäßen Vorrichtung ist die Prüfeinrichtung bzw. sind mindestens Teile der Prüfeinrichtung in den HF-Generator zum Erzeugen einer hochfrequenten Spannung integriert ausgebildet. Das heißt, der HF-Generator ist derart ausgebildet, dass bei Anstecken der Neutralelektrode an den Generator ein Erkennungsprozedere durchführbar ist, ohne dass hierfür eine eigene Einrichtung notwendig wäre. Ganz allgemein lässt sich die Prüfeinrichtung in ein HF-Chirurgiegerät einer HF-chirurgischen Einrichtung integrieren.

Eine Ausführungsform sieht vor, dass die Prüfeinrichtung derart ausgebildet ist, dass sie den HF-Generator aufgrund der erfassten Kodierung, also z. B. aufgrund des erfassten Widerstandswertes zu dessen Einstellung ansteuert. Das heißt, etwaige am HF-Generator einzustellende Werte, wie z. B. die Stromstärke, würden aufgrund der erkannten Neutralelektrode automatisch eingestellt werden. Dies ist insbesondere dann von Vorteil, wenn Neutralelektroden im Einsatz sind, die bei Überschreiten einer bestimmten Stromstärke Verbrennungen des Patienten verursachen würden. So könnte man insbesondere bei Neutralelektroden für Säuglinge und Kleinkinder automatisch eine geeignete Strombegrenzung aktivieren.

Möglich ist es auch, dass der Prüfeinrichtung eine Steuerungseinrichtung zugeordnet ist (die ggf. auch in die Prüfeinrichtung integriert ist), die derart ausgebildet ist, dass sie den HF-Generator aufgrund der erfassten Kodierung bzw. des erfassten Widerstandswertes zu dessen Einstellung ansteuert. Eine explizite Steuerungseinrichtung könnte hierfür auch programmierbar ausgebildet sein und so die Steuerung bzw. Regelung des HF-Generators übernehmen.

Möglich ist es natürlich auch, die entsprechenden Einstellungen aufgrund des erkannten Widerstandswertes per Hand vorzunehmen. Sobald der Operateur die Rückmeldung über die erkannte Neutralelektrode vom System erhält, kann er die erforderlichen Einstellungen, insbesondere am HF-Generator, vornehmen.

Vorzugsweise ist der Prüfeinrichtung eine Speichereinrichtung zugeordnet, in der Kodierungen, also z. B. Widerstandswerte von Widerstandselementen, von verschiedenen Neutralelektroden als Vergleichswerte zur Neutralelektrodenerkennung speicherbar sind. Damit können auf einfache Weise Angaben über die verwendete Neutralelektrode ausgegeben werden, was dem Benutzer die Zuordnung erleichtert und den Verlauf des Eingriffs planbar macht. So kann der Operateur.z. B. entsprechende Einstellungen am HF-Generator vornehmen, die an die verwendete Neutralelektrode angepasst sind

Die Angaben über die identifizierte Neutralelektrode können z. B. über ein Display am HF-Generator bzw. an der HF-Chirurgieeinrichtung ganz allgemein ausgegeben werden. Auch lassen sich Töne, Lichtsignale oder dergleichen Signale hierfür einsetzen.

Vorzugsweise ist der Prüfeinrichtung eine Eingabeeinheit zugeordnet, welche derart ausgebildet ist, dass ein Anwender z. B. die Vergleichswerte in die Speichereinrichtung eingeben kann. Auch jegliche sonstige Kommunikation mit der HF-Chirurgieeinrichtung ist über die Eingabeeinheit, z. B. eine Tastatur, möglich. Die Speichereinrichtung kann auch derart ausgebildet sein, dass noch nicht gespeicherte Kodierungen bzw. Werte etwaiger Widerstände von Neutralelektroden erfasst und gespeichert werden, sobald diese durch die Messeinrichtung erfasst sind.

Nachfolgend wird die Erfindung anhand von Ausführungsbeispielen beschrieben, die anhand der Abbildungen näher erläutert werden. Hierbei zeigen:
- - Fig. 1: eine Ausführungsform der erfindungsgemäßen Prüfeinrichtung;
- - Fig. 2: eine weitere Darstellung gemäß der Ausführungsform nach Fig. 1;
- - Fig. 3: eine weitere Darstellung gemäß der Ausführungsform nach Fig. 1.

In der nachfolgenden Beschreibung werden für gleiche und gleich wirkende Teile dieselben Bezugsziffern verwendet.

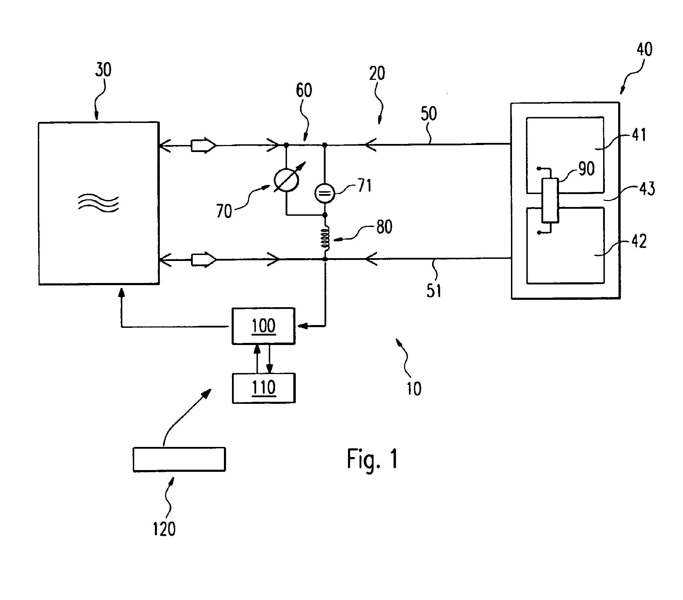

In Fig. 1 ist eine Ausführungsform einer erfindungsgemäßen Prüfeinrichtung 20 gezeigt. An einem einen hochfrequenten Strom liefernden HF-Generator 30 einer HF-Chirurgieeinrichtung 10 ist eine zweiteilige Neutralelektrode 40 angeschlossen. Die Neutralelektrode 40 weist einen ersten und einen zweiten elektrisch leitfähigen Elektrodenabschnitt 41, 42 auf, wobei der erste Elektrodenabschnitt 41 über eine erste Leitung 50 mit dem HF-Generator 30 verbunden ist und der zweite Elektrodenabschnitt 42 über eine zweite Leitung 51. Die Elektrodenabschnitte sind gemeinsam auf einem Trägerelement 43 angeordnet.

Die Neutralelektrode 40 ist als indifferente Elektrode insbesondere bei monopolaren Behandlungsmethoden auf einen Gewebeabschnitt eines Patienten aufbringbar und dient letztendlich der Stromableitung über einen relativ großen Flächenbereich. Die flächige Ausgestaltung der Elektrodenabschnitte 41, 42 gewährleistet eine gute Stromverteilung, so dass punktuell keine hohen Stromspitzen beim Übergang zwischen Gewebe und Neutralelektrode 40 auftreten. Damit lassen sich Verbrennungen und dergleichen Verletzungen des Patienten vermeiden.

Vorteilhaft ist es, wenn die Neutralelektrode 40 für ihren Einsatz identifizierbar ist. Das heißt, sind wesentliche Parameter der Neutralelektrode 40 für den Operateur erkennbar, so lassen sich etwaige Einstellung bzgl. Stromstärke etc. am HF-Generator 30 gezielt auf die Neutralelektrode 40 abgestimmt vornehmen. Dabei kann die Einstellung z. B. durch den Operateur per Hand erfolgen, oder aber die Prüfeinrichtung 20 ist derart ausgebildet oder derart mit einer Steuerungseinrichtung 100 verbunden, dass notwendige Einstellungen automatisch erfolgen. Die Erkennung der Neutralelektrode 40 und damit verbundene Abstimmungen von Einstellungsparametern sind z. B. wichtig für Elektroden zum Gebrauch bei Säuglingen und Kleinkindern. So kann z. B. bei diesen Elektroden aufgrund ihrer Identifizierung eine passende Strombegrenzung selbsttätig (oder auch per Hand) eingestellt werden.

Zur Identifizierung der Neutralelektrode 40 ist bei diesem Ausführungsbeispiel ein Widerstandselement 90 als ein Kodierungselement zwischen den beiden Elektrodenabschnitten 41, 42 vorgesehen. Das Widerstandselement 90 weist einen bestimmten Widerstandswert als Kodierung auf, der bzw. die für die entsprechende Neutralelektrode 40 charakteristisch ist, so dass der genaue Typ der verwendeten Neutralelektrode bestimmbar ist. Das Widerstandselement 90 ist hier zwischen die beiden leitfähigen Elektrodenabschnitte 41, 42 geschaltet.

Zur Erfassung des Widerstandswertes sind Komponenten der Prüfeinrichtung 20 zwischen Generator 30 und Neutralelektrode 40 geschaltet. Die Prüfeinrichtung 20 umfasst hier neben dem Widerstandselement eine Gleichstromquelle als Messstromquelle 71, eine Spannungsmesseinrichtung 70 zum indirekten Erfassen des Widerstandswertes des Widerstandselements 90 und eine Induktivität, also eine Spule als Filterelement 80, die die Entkopplung von Arbeitsstrom und Prüf- bzw. Messstrom ermöglicht. Diese Komponenten der Prüfeinrichtung 20 bilden letztendlich eine Messeinrichtung 60 aus und sind derart angeordnet, dass der Messstrom über die ohnehin vorhandenen Leitungen zum Verbinden der Neutralelektrode 40 mit dem HF-Generator 30 führbar ist. Die Prüfeinrichtung umfasst also das Kodierungselement 90 und die Messeinrichtung 60.

Der Messstrom ist als Gleichstrom oder als niederfrequenter Wechselstrom vom hochfrequenten Arbeitsstrom entkoppelbar, indem z. B. die Spule als Filter vorgesehen ist, deren Blindwiderstand umso größer ist, je höher die Frequenz ist.

Der Gleichstrom wird von der Gleichstromquelle 71 geliefert; die Messung des Widerstandswertes erfolgt z. B. indirekt über die Spannungsmesseinrichtung 70 mit anschlie-βender Widerstandsberechnung. Auch könnten z. B. Messbrücken zur Widerstandsbestimmung verwendet werden.

Mittels des Gleichstroms oder auch eines niederfrequenten Wechselstroms (Messstrom) kann der Widerstand vermessen werden, ohne dass der Messstrom über den Patientenkörper fließt. Der Patientenkörper koppelt im Wesentlichen nur kapazitiv an die Elektrode. Eine gesonderte Anschlussleitung zur Signalübertragung ist hier nicht nötig. Es wird also ein Strom aufgeprägt, der mangels Kopplung des Patientenkörpers nicht als Arbeitsstrom verwendet werden kann und somit eine Elektrodenerkennung ermöglicht, ohne eine dafür gesonderte Übertragungsleitung zu benötigen. Ein auf den Elektroden 41, 42 angebrachtes Hydrogel 44, 44' zum Kontaktieren der Neutralelektrode 40 mit dem Gewebe 130 des Patienten weist hierzu eine chemische Zusammensetzung derart auf, dass es für Gleichstrom oder Wechselstrom mit niedrigen Frequenzen (bis ca. 100 Hz) einen hochohmigen Widerstand R_{Gel} und R'_{Gel} darstellt. Da sich das Gel im Bereich des Messstromes hochohmig verhält, ist es unerheblich, ob die Neutralelektrode am Patienten angelegt ist, d. h. ein niederohmiger Parallelwiderstand Rₚ durch das Gewebe 130 vorhanden ist oder nicht. Durch eine Art sowieso vorhandenen Filters (kapazitives Ankoppeln der Neutralelektrode an den Patienten) und Einsetzen eines "ungeeigneten" Messstroms sind zusätzliche Leitungen für die Datenübertragung im Rahmen der Neutralelektrodenerkennung nicht erforderlich.

Gegebenenfalls kann - wie bereits oben beschrieben - die Steuerungseinrichtung 100 vorgesehen werden, über welche der HF-Generator 30 aufgrund des erfassten Kodierungswertes, also z. B. aufgrund des erfassten Widerstandswertes ansteuerbar ist. Der HF-Generator 30 ist dann z. B. auf einen bestimmten Stromwert einstellbar oder es wird eine Strombegrenzung vorgegeben. Dies ist insbesondere bei Neutralelektroden für Kinder von Vorteil, um eine Überhitzung zu vermeiden.

Die Steuerungseinrichtung 100 kann integral zumindest mit Teilen der Prüfeinrichtung 20 ausgebildet werden und Prüfeinrichtung und/oder Steuerungseinrichtung können auch integral mit dem HF-Generator 30 ausgebildet sein. In diesem Ausführungsbeispiel ist der Steuerungseinrichtung 100 zudem eine Speichereinrichtung 110 zugeordnet (die auch unmittelbar der Prüfeinrichtung 20 zugeordnet werden könnte). So kann jedem Neutralelektroden-Modell ein bestimmter Widerstandswert als Kodierung zugewiesen werden. Durch eine in der Speichereinrichtung 110 abgelegte Tabelle (Art der Neutralelektrode - zugehörige Einstellungsparameter) kann insbesondere der HF-Generator 30 im Rahmen einer Instrumenten- (bzw. Elektroden-) orientierten Systemkonfiguration selbsttätig auf die Verhältnisse an der erkannten Neutralelektrode eingestellt werden.

Ferner ist der Prüfeinrichtung 20 hier eine Eingabeeinheit 120 zugeordnet, über welche ein Anwender mit dem System kommunizieren, z. B. einzuspeichernde Informationen eingeben kann.

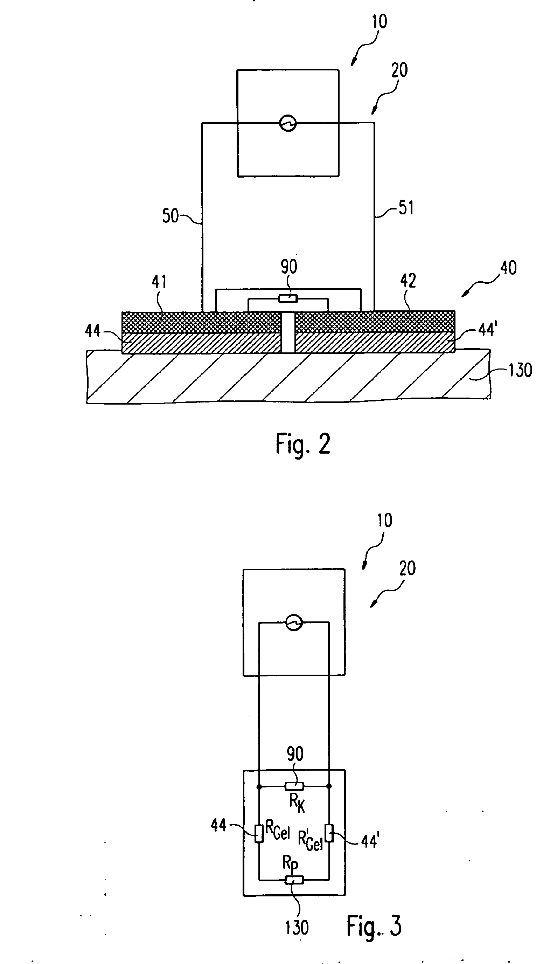

Die Fig. 2 und 3 zeigen die Anordnung gemäß Fig. 1 noch einmal in anderer Darstellungsweise. Fig. 2 zeigt die auf einem Gewebeabschnitt 130 eines Patienten mittels Hydrogels 44, 44' aufgebrachte Neutralelektrode 40. Die beiden leitfähigen Elektrodenabschnitte 41, 42 sind mittels eines Spalts voneinander getrennt. Verbunden sind die beiden Elektrodenabschnitte über das Kodierungselement, dem Widerstandselement 90 mit dem für die Neutralelektrode 40 charakteristischen Widerstandswert. Über die Anschlusskabel der Elektrodenabschnitte (Leitungen) 50, 51 zum Verbinden der Elektrodenabschnitte 41, 42 mit dem HF-Generator 30 lässt sich der Messstrom über die Messeinrichtung 60 derart aufprägen, dass der Widerstandswert erfasst, also gemessen werden kann. Der erste Elektrodenabschnitt 41 und der zweite Elektrodenabschnitt 42 liegen über das Gel 44, 44' auf dem Gewebe 130 des Patienten auf. Vereinfacht ist das Neutralelektroden-Überwachungssystem dargestellt, das z. B. in den HF-Generator 30 bzw. in ein HF-Chirurgiegerät einer HF-chirurgischen Einrichtung 10 integriert ist. Die zweigeteilte aktive Kontaktfläche (Elektrodenabschnitte) ist z. B. aus Aluminium ausgebildet.

Fig. 3 zeigt als Ersatzschaltbild die einzelnen Widerstände, wie sie zueinander geschaltet sind. Das Kodierungselement 90, d. h. das Widerstandselement mit dem Widerstand R_{K} ist parallel zu einem Patientenwiderstand Rₚ geschaltet. Die beiden Widerstände R_{Gel} und R'_{Gel} der Gelschichten 44 und 44' unter den jeweiligen Elektrodenabschnitten zeigen bei Gleichstrom oder niederfrequentem Messstrom ein hochohmiges Verhalten, wie oben bereits beschrieben.

Es lässt sich festhalten, dass zur Erfassung einer für die Neutralelektrode charakteristischen Kodierung die Leitungen verwendbar sind, mit welchen die Neutralelektrode an den HF-Generator angeschlossen wird. Durch den geeigneten Messstrom können die Parameter der Messschaltung derart vorgegeben werden, dass mit geringstem Aufwand eine Neutralelektrodenerkennung durchführbar ist.

### Bezugszeichenliste

- 10: HF-Chirurgieeinrichtung
- 20: HF-chirurgische Prüfeinrichtung
- 30: HF-Generator
- 40: Neutralelektrode
- 41: Erster Elektrodenabschnitt
- 42: Zweiter Elektrodenabschnitt
- 43: Trägerelement
- 44, 44': Gelschicht
- 50: Erste Leitung
- 51: Zweite Leitung
- 60: Messeinrichtung .
- 70: Spannungsmesseinrichtung
- 71: Messstromquelle
- 80: Filterelement, Spule
- 90: Kodierungselement, Widerstandselement
- 100: Steuerungseinrichtung
- 110: Speichereinrichtung
- 120: Eingabeeinheit
- 130: Gewebe
- R_{K}: Widerstand Kodierungselement, Widerstandselement
- R_{Gel}, R'_{Gel}: Widerstand Gel
- Rₚ: Widerstand Patient

## Patentansprüche

1. HF-chirurgische Prüfeinrichtung zum Prüfen einer Neutralelektrode (40) beim Behandeln, insbesondere monopolaren Koagulieren biologischen Gewebes (130) mittels eines hochfrequenten Stromes,
**dadurch gekennzeichnet, dass**
die Prüfeinrichtung (20) eine Neutralelektrode (40) umfasst, die
- mindestens einen ersten, mit dem Gewebe (130) in Kontakt bringbaren elektrisch leitfähigen Elektrodenabschnitt (41) mit einer ersten Leitung (50) zum Verbinden mit einem HF-Generator (30)
- und einen zweiten, mit dem Gewebe (130) in Kontakt bringbaren elektrisch leitfähigen Elektrodenabschnitt (42) mit einer zweiten Leitung (51) zum Verbinden mit dem HF-Generator (30) umfasst,
wobei die Prüfeinrichtung (20) ein Kodierungselement (90) mit einer die Neutralelektrode (40) charakterisierenden Kodierung zwischen dem ersten und dem zweiten Elektrodenabschnitt (41, 42) und eine Messeinrichtung (60) umfasst, die derart ausgebildet ist, dass die Kodierung zur Erkennung der Neutralelektrode (40) erfassbar ist.

2. HF-chirurgische Prüfeinrichtung nach Anspruch 1,
**dadurch gekennzeichnet, dass**
die Prüfeinrichtung (20) eine Quelle (71) für Gleichstrom oder niederfrequenten Wechselstrom als Prüfstrom zur Erfassung der Kodierung des Kodierungselements (90) umfasst.

3. HF-chirurgische Prüfeinrichtung nach Anspruch 1 oder 2,
**dadurch gekennzeichnet, dass**
die Prüfeinrichtung (20) derart zwischen der ersten Leitung und der zweiten Leitung angeordnet ist, dass der Prüfstrom über die erste und zweite Leitung (50, 51) führbar ist.

4. HF-chirurgische Prüfeinrichtung nach einem der vorhergehenden Ansprüche,
**dadurch gekennzeichnet, dass**
das Kodierungselement (90) ein Widerstandselement mit einem die Neutralelektrode (40) charakterisierenden Widerstandswert umfasst, wobei die Prüfeinrichtung (20) derart ausgebildet ist, dass der Widerstandswert zur Erkennung der Neutralelektrode (40) erfassbar ist.

5. HF-chirurgische Prüfeinrichtung nach Anspruch 4,
**dadurch gekennzeichnet, dass**
das Widerstandselement (90) als ein ohmscher Widerstand oder ein komplexer Widerstand mit induktivem Verhalten ausgebildet ist.

6. HF-chirurgische Prüfeinrichtung nach Anspruch 4 oder 5,
**dadurch gekennzeichnet, dass**
das Widerstandselement (90) als eine in die Neutralelektrode integrierte Widerstandsfolie oder ein Widerstandsdraht vorgesehen ist.

7. HF-chirurgische Prüfeinrichtung nach Anspruch 4 oder 5,
**dadurch gekennzeichnet, dass**
das Widerstandselement (90) zwischen der ersten Leitung (50) und der zweiten Leitung (51), vorzugsweise an einer der Leitungen, angeordnet ist.

8. HF-chirurgische Prüfeinrichtung nach einem der Ansprüche 4 bis 7,
**dadurch gekennzeichnet, dass**
die Prüfeinrichtung (20) eine Spannungsmesseinrichtung (70) zur Messung einer über dem Widerstandselement (90) abfallenden Spannung aufweist.

9. HF-chirurgische Prüfeinrichtung nach einem der vorhergehenden Ansprüche,
**dadurch gekennzeichnet, dass**
die Prüfeinrichtung (20) eine Strommesseinrichtung zur Messung des Gleichstroms oder des niederfrequenten Wechselstroms umfasst.

10. HF-chirurgische Prüfeinrichtung nach einem der vorhergehenden Ansprüche,
**dadurch gekennzeichnet, dass**
mindestens Teile der Prüfeinrichtung (20) in den HF-Generator (30) zum Erzeugen einer hochfrequenten Spannung bzw. in einem HF-Chirurgiegerät integriert ausgebildet sind.

11. HF-chirurgische Prüfeinrichtung nach einem der Ansprüche 4 bis 10,
**dadurch gekennzeichnet, dass**
die Prüfeinrichtung (20) derart ausgebildet ist, dass sie den HF-Generator (30) aufgrund des erfassten Widerstandswertes zu dessen Einstellung ansteuert.

12. HF-chirurgische Prüfeinrichtung nach einem der Ansprüche 4 bis 11,
**dadurch gekennzeichnet, dass**
die Prüfeinrichtung (20) eine Steuerungseinrichtung (100) aufweist, die derart ausgebildet ist, dass sie den HF-Generator (30) aufgrund des erfassten Widerstandswertes zu dessen Einstellung ansteuert.

13. HF-chirurgische Prüfeinrichtung nach einem der Ansprüche 4 bis 12,
**dadurch gekennzeichnet, dass**
die Prüfeinrichtung eine Speichereinrichtung (110) aufweist, in der Widerstandswerte von Widerstandselementen von verschiedenen Neutralelektroden als Vergleichswerte zur Neutralelektrodenerkennung speicherbar sind.

14. HF-chirurgische Prüfeinrichtung nach Anspruch 13,
**dadurch gekennzeichnet, dass**
die Prüfeinrichtung (20) eine Eingabeeinheit (120) aufweist, welche derart ausgebildet ist, dass ein Anwender insbesondere die Vergleichswerte in die Speichereinrichtung (110) eingeben kann.

## Claims

1. Electrosurgical testing apparatus for testing a neutral electrode (40) during treatment, more particularly monopolar coagulation of biological tissue (130) by means of radiofrequency current, **characterized in that**
the testing apparatus (20) comprises a neutral electrode (40), which
- comprises at least one first electrically conductive electrode section (41), which can be brought into contact with the tissue (130) and has a first line (50) for connection to an RF generator (30) and
- a second electrically conductive electrode section (42), which can be brought into contact with the tissue (130) and has a second line (51) for connection to the RF generator (30),
the testing apparatus (20) comprising an encoding element (90) with a code characterizing the neutral electrode (40), between the first and the second electrode section (41, 42) and a measuring apparatus (60), the latter being embodied such that the code can be detected for identifying the neutral electrode (40).

2. Electrosurgical testing apparatus according to Claim 1,
**characterized in that**
the testing apparatus (20) comprises a source (71) for direct current or low-frequency alternating current as testing current for detecting the code of the encoding element (90).

3. Electrosurgical testing apparatus according to Claim 1 or 2,
**characterized in that**
the testing apparatus (20) is arranged between the first line and the second line such that the testing current can be routed over the first and second lines (50, 51).

4. Electrosurgical testing apparatus according to one of the preceding claims,
**characterized in that**
the encoding element (90) comprises a resistance element with a resistance value characterizing the neutral electrode (40), with the testing apparatus (20) being embodied such that the resistance value can be detected for identifying the neutral electrode (40).

5. Electrosurgical testing apparatus according to Claim 4,
**characterized in that**
the resistance element (90) is embodied as ohmic resistance or as a complex impedance with inductive behaviour.

6. Electrosurgical testing apparatus according to Claim 4 or 5,
**characterized in that**
the resistance element (90) is provided as a resistance film integrated into the neutral electrode or as a resistance wire.

7. Electrosurgical testing apparatus according to Claim 4 or 5,
**characterized in that**
the resistance element (90) is arranged between the first line (50) and the second line (51), preferably on one of the lines.

8. Electrosurgical testing apparatus according to one of Claims 4 to 7,
**characterized in that**
the testing apparatus (20) has a voltage measuring apparatus (70) for measuring a voltage which drops over the resistance element (90).

9. Electrosurgical testing apparatus according to one of the preceding claims,
**characterized in that**
the testing apparatus (20) comprises a current measuring apparatus for measuring the direct current or the low-frequency alternating current.

10. Electrosurgical testing apparatus according to one of the preceding claims,
**characterized in that**
at least parts of the testing apparatus (20) are embodied integrated into the RF generator (30) for generating a radiofrequency voltage or into an electrosurgical device.

11. Electrosurgical testing apparatus according to one of Claims 4 to 10,
**characterized in that**
the testing apparatus (20) is embodied such that it actuates the RF generator (30) for setting the latter on the basis of the detected resistance value.

12. Electrosurgical testing apparatus according to one of Claims 4 to 11,
**characterized in that**
the testing apparatus (20) has a control apparatus (100) which is embodied such that it actuates the RF generator (30) for setting the latter on the basis of the detected resistance value.

13. Electrosurgical testing apparatus according to one of Claims 4 to 12,
**characterized in that**
the testing apparatus has a storage apparatus (110), in which resistance values of resistance elements of various neutral electrodes can be stored as comparison values for neutral electrode identification.

14. Electrosurgical testing apparatus according to Claim 13,
**characterized in that**
the testing apparatus (20) has an input unit (120) which is embodied such that a user can enter the comparison values in particular into the storage apparatus (110).

## Revendications

1. Dispositif de contrôle chirurgical HF pour contrôler une électrode neutre (40) lors du traitement, notamment de la coagulation monopolaire d'un tissu biologique (130) au moyen d'un courant à haute fréquence,
**caractérisé en ce que**
le dispositif de contrôle (20) comprend une électrode neutre (40) qui inclut
- au moins une première portion d'électrode (41) électriquement conductrice pouvant être mise en contact avec le tissu (130) et munie d'une première ligne (50) pour être reliée avec un générateur HF (30),
- et une deuxième portion d'électrode (42) électriquement conductrice pouvant être mise en contact avec le tissu (130) et munie d'une deuxième ligne (51) pour être reliée avec le générateur HF (30),
le dispositif de contrôle (20) incluant un élément de codage (90) qui présente un codage caractérisant l'électrode neutre (40) entre la première et la deuxième portion d'électrode (41, 42) et un dispositif de mesure (60) qui est configuré de telle sorte que le codage peut être détecté pour identifier l'électrode neutre (40).

2. Dispositif de contrôle chirurgical HF selon la revendication 1,
**caractérisé en ce que**
le dispositif de contrôle (20) comprend une source (71) de courant continu ou de courant alternatif à basse fréquence pour la détection du codage de l'élément de codage (90).

3. Dispositif de contrôle chirurgical HF selon la revendication 1 ou 2,
**caractérisé en ce que**
le dispositif de contrôle (20) est disposé entre la première ligne et la deuxième ligne de telle sorte que le courant de contrôle peut être acheminé par le biais de la première et de la deuxième ligne (50, 51).

4. Dispositif de contrôle chirurgical HF selon l'une des revendications précédentes,
**caractérisé en ce que**
l'élément de codage (90) comprend un élément résistif ayant une valeur de résistance qui caractérise l'électrode neutre (40), le dispositif de contrôle (20) étant configuré de telle sorte que la valeur de résistance peut être détectée pour identifier l'électrode neutre (40).

5. Dispositif de contrôle chirurgical HF selon la revendication 4,
**caractérisé en ce que**
l'élément résistif (90) est réalisé sous la forme d'une résistance ohmique ou d'une résistance plus complexe ayant un comportement inductif.

6. Dispositif de contrôle chirurgical HF selon la revendication 4 ou 5,
**caractérisé en ce que**
l'élément résistif (90) est prévu sous la forme d'un film résistif intégré dans l'électrode neutre ou d'un fil résistif.

7. Dispositif de contrôle chirurgical HF selon la revendication 4 ou 5,
**caractérisé en ce que**
l'élément résistif (90) est disposé entre la première ligne (50) et la deuxième ligne (51), de préférence sur l'une des lignes.

8. Dispositif de contrôle chirurgical HF selon l'une des revendications 4 à 7,
**caractérisé en ce que**
le dispositif de contrôle (20) présente un dispositif de mesure de tension (70) pour mesurer une chute de tension aux bornes de l'élément résistif (90).

9. Dispositif de contrôle chirurgical HF selon l'une des revendications précédentes,
**caractérisé en ce que**
le dispositif de contrôle (20) comprend un dispositif de mesure du courant pour mesurer le courant continu ou le courant alternatif à basse fréquence.

10. Dispositif de contrôle chirurgical HF selon l'une des revendications précédentes,
**caractérisé en ce**
**qu'**au moins des parties du dispositif de contrôle (20) sont réalisées intégrées dans le générateur HF (30) pour générer une tension à haute fréquence ou dans un appareil chirurgical HF.

11. Dispositif de contrôle chirurgical HF selon l'une des revendications 4 à 10,
**caractérisé en ce que**
le dispositif de contrôle (20) est configuré de telle sorte qu'il commande le générateur HF (30) en se basant sur la valeur de résistance détectée pour son réglage.

12. Dispositif de contrôle chirurgical HF selon l'une des revendications 4 à 11,
**caractérisé en ce que**
le dispositif de contrôle (20) présente un dispositif de commande (100) qui est configuré de telle sorte qu'il commande le générateur HF (30) en se basant sur la valeur de résistance détectée pour son réglage.

13. Dispositif de contrôle chirurgical HF selon l'une des revendications 4 à 12,
**caractérisé en ce que**
le dispositif de contrôle présente un dispositif de mémorisation (110) dans lequel peuvent être enregistrées les valeurs de résistance des éléments résistifs de différentes électrodes neutres en tant que valeurs comparatives pour l'identification de l'électrode neutre.

14. Dispositif de contrôle chirurgical HF selon la revendication 13,
**caractérisé en ce que**
le dispositif de contrôle (20) présente une unité de saisie (120) qui est configurée de telle sorte qu'un utilisateur peut notamment saisir les valeurs comparatives dans le dispositif de mémorisation (110).
